Europäisches Patentamt

European Patent Office

Office européen des brevets

(19)

(11) Numéro de publication : **0 449 690 A1**

## (12) DEMANDE DE BREVET EUROPEEN

(21) Numéro de dépôt : **91400663.0**

(22) Date de dépôt : **11.03.91**

(51) Int. Cl.$^5$ : **G01T 1/29**, G01T 1/202

(30) Priorité : **12.03.90 FR 9003101**

(43) Date de publication de la demande :
**02.10.91 Bulletin 91/40**

(84) Etats contractants désignés :
**DE FR GB SE**

(71) Demandeur : **COMMISSARIAT A L'ENERGIE ATOMIQUE**
**31-33, rue de la Fédération**
**F-75015 Paris (FR)**

(72) Inventeur : **Monnet, Olivier**
**6 Avenue M. Chorot**
**F-38430 Moirans (FR)**
Inventeur : **Vacher, Jacques**
**64 Avenue du Vercors**
**F-38170 Seyssinet (FR)**

(74) Mandataire : **Mongrédien, André et al**
**c/o BREVATOME 25, rue de Ponthieu**
**F-75008 Paris (FR)**

(54) **Dispositif pour la visualisation de désintégrations de positons.**

(57)     Dispositif pour la visualisation de désintégrations de positons comportant au moins une paire de scintillateurs (28) sensibles aux rayonnements gamma et disposés en regard l'un de l'autre, des ensembles de photomultiplicateurs (30) associés chacun à un scintillateur, des moyens (34) de mesure barycentrique de la position des scintillations dans les scintillateurs, des moyens (36) de détermination des temps de vol des rayonnements gamma détectés en coïncidence dans chacun des scintillateurs appariés, des moyens (38) de localisation des désintégrations de positons et un système (40) de visualisation d'image.
    Application à l'étude d'organes inaccessibles vivants ou non.

EP 0 449 690 A1

FIG. 4

## DISPOSITIF POUR LA VISUALISATION DE DESINTEGRATIONS DE POSITONS

La présente invention a pour objet un dispositif pour la visualisation de désintégrations de positons. Elle s'applique notamment en médecine, pour les études de métabolismes, en pharmacologie, pour suivre le comportement d'un médicament dans l'organisme, et dans l'industrie, pour l'étude en fonctionnement de pièces mettant en jeu des fluides en mouvement.

On connaît un dispositif pour la visualisation de désintégrations de positons : le tomographe à positons qui utilise la propriété qu'ont les positons de se désintégrer lors d'une rencontre avec un électron en émettant deux rayonnements gamma dirigés selon deux directions opposées et possèdant chacun une énergie de 511 keV.

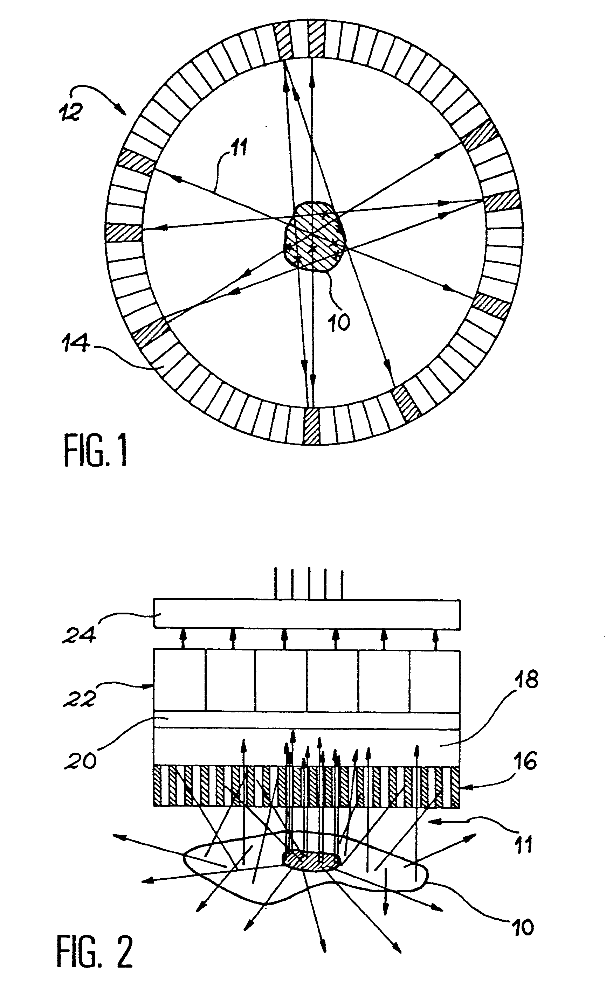

La figure 1 représente schématiquement une vue en coupe d'un tomographe à positons.

Lors d'une étape préliminaire, on injecte une substance contenant des éléments radioactifs émettant des positons dans l'organe à visualiser 10 (ici, un cerveau, donc de matière vivante mais cet organe peut-être mécanique). L'organe 10 est placé au centre d'une couronne 12 de détecteurs 14 formés chacun d'un scintillateur sensible aux rayonnements gamma couplé à un photomultiplicateur. Chaque positon émis se désintègre avec un électron après un libre parcours moyen dépendant de l'organe 10 étudié (ce libre parcours moyen est de 2 à 3 mm dans le corps humain). Une désintégration donne lieu à une émission de deux rayonnements gamma 11 de directions opposées qui sont détectés en coïncidence par les détecteurs 14 de la couronne. Ces détections en coïncidence réalisent une collimation électronique qui permet de matérialiser la trace des rayonnements gamma couplés.

Ensuite, par un traitement de reconstruction d'image faisant appel à des opérations telles que des rétroprojections des traces selon plusieurs directions, des convolutions ..., on peut localiser les points de désintégration des positons.

On comprend que cette visualisation n'est obtenue que pour une tranche de l'organe 10 située dans le plan de la couronne 12 de détecteurs 14.

Pour obtenir une visualisation en volume, on juxtapose plusieurs couronnes 12 les unes à côté des autres.

De manière connue, on améliore la sensibilité des tomographes à positons en effectuant une mesure complémentaire dite mesure des temps de vol des rayonnements gamma qui permet de localiser chaque point de désintégration d'un positon sur la trace correspondante. Le temps de vol correspond à la différence de temps d'arrivée de deux rayonnements gamma détectés en coïncidence. La connaissance de la position des détecteurs 14 activés obtenue par la détection des traces, de la vitesse de la lumière et du temps de vol permet de localiser le lieu d'émission des rayonnements gamma. En fait, la résolution temporelle des détecteurs 14 et de l'électronique d'analyse associée ne permet qu'une résolution spatiale de 5 cm environ dans l'état actuel de la technique. La localisation obtenue par ce moyen n'est donc pas suffisamment précise pour dresser une cartographie des points de désintégration ; elle permet simplement de restreindre la plage de reconstruction à la portion de trace située dans la zone délimitée par le temps de vol et par conséquent de ne pas prendre en compte le bruit apparaissant hors de cette zone. La tomographie à temps de vol à cause de sa résolution temporelle insuffisante ne permet donc pas de se passer des techniques de reconstruction d'image.

Par ailleurs, dans un tomographe à positons usuel constitué d'une ou plusieurs couronnes de détecteurs discrets, on sait que la résolution spatiale au centre du dispositif est sensiblement égale à la demi-largeur d'un détecteur. Or, une trace est séparée de sa voisine par le pas entre deux détecteurs, lequel est évidemment au moins égal à une largeur de détecteur. Il s'ensuit que l'information recueillie est sous échantillonnée. Pour pallier cet inconvénient, la ou les couronnes sont animées d'un mouvement de rotation alternatif et de faible amplitude qui permet à chaque détecteur d'occuper toutes les positions intermédiaires entre sa position au repos et celles de ses voisins. Ce petit mouvement circulaire, d'un rayon égal à un demi-pas entre détecteurs, est appelé communément brimbalement ("Wobbling" en terminologie anglo-saxonne).

Ce brimbalement, nécessaire au bon fonctionnement des tomographes à positons, entraîne une complexité mécanique importante qui rend la mise en oeuvre du dispositif difficile tout en entraînant des surcoûts importants.

On connaît par ailleurs un dispositif : "la gamma caméra" en terminologie anglo-saxonne SPECT (Single Photon Emission Computed Tomography) permettant la visualisation de rayonnements gamma émis par un organe rendu radio-émissif par l'injection d'un traceur gamma qui ne produit qu'un rayonnement gamma par désintégration. On trouve une description de "gamma caméra" dans le brevet US n° 3 011 057. Contrairement aux tomographes à positons qui réalisent une collimation électronique, les "gamma caméras" utilisent un collimateur matériel 16. Ce dernier est formé d'une plaque en matériau absorbant par exemple en plomb percée de trous orientés soit parallèlement entre eux et perpendiculaires à la surface du scintillateur 18 auquel le collimateur 16 est accolé soit dans une direction convergeant vers le centre du dispositif pour obtenir un effet d'agrandissement. La taille et l'orientation des trous sont telles que seuls les rayonnements gamma possédant une direction de propagation correspondante sont transmis.

Les rayonnements gamma interagissant avec le scintillateur 18 provoquent des scintillations qui sont détectées par plusieurs tubes photomultiplicateurs 22 disposés côte à côte sur la face arrière du scintillateur. Les tubes photomultiplicateurs sont éloignés de la face arrière du scintillateur 18 par une couche 20 de matériau transparent.

Si le scintillateur 18 utilisé était en contact direct avec les photomultiplicateurs, une scintillation intervenant au centre de la zone de détection d'un photomulplicateur et très près de celui-ci pourrait être difficilement détectée par d'autres photomultiplicateurs sans cette couche 20 de matériau transparent. Or, il est important que chaque scintillation soit détectée par plusieurs photomultiplicateurs 22.

En effet, une mesure barycentrique de la position de chaque scintillation est effectuée par des moyens électroniques 24 à l'aide des signaux proportionnels à l'intensité lumineuse détectée, délivrés par les tubes photomultiplicateurs 22.

Ces moyens électroniques 24 de mesure barycentrique connectés aux sorties des photomultiplicateurs 22 délivrent des signaux indiquant la position moyenne de chaque scintillation sur le scintillateur 18. Cette position est définie uniquement selon deux directions contenues dans un plan parallèle à la face de couplage entre scintillateur et photomultiplicateur.

Pour obtenir une image en trois dimensions de l'organe, la gamma caméra tourne autour de l'organe : on détecte ainsi toutes les directions de rayonnements gamma.

L'ensemble des directions est traité par un système de reconstruction d'image et de visualisation (non représenté) qui permet d'obtenir une image des points émettant un rayonnement gamma dans l'organe étudié.

A cause de la nécessité d'obtenir des rayonnements gamma de directions déterminées avant qu'ils n'interagissent avec le scintillateur 18, le collimateur 16 doit présenter des trous de diamètre extrêmement réduit. La quantité de rayonnements gamma donnant lieu à des scintillations est donc faible. En d'autres termes, la sensibilité d'une "gamma caméra" est faible, ce qui est l'inconvénient majeur de ce type de dispositif. En effet, plus faible est la sensibilité du détecteur, plus forte doit être la dose d'élément radioactif injectée dans l'organe. Et cette dose est limitée lorsqu'un organe vivant est à marquer.

Dans les tomographes à positons comme dans les gamma caméras, pour obtenir une image en trois dimensions, de résolution acceptable, il est nécessaire d'effectuer un traitement de reconstruction d'image, ce qui alourdit considérablement la complexité des dispositifs.

Un premier but de la présente invention est de fournir un dispositif pour la visualisation de désintégration de positons ne nécessitant pas de traitement de reconstruction d'image pour obtenir une résolution acceptable. Bien entendu, ce traitement de reconstruction d'image peut être utilisé dans le dispositif de l'invention pour améliorer sa résolution.

Un second but de la présente invention est de fournir un dispositif suffisamment sensible pour que la dose d'élément radioactif nécessaire pour marquer un organe soit faible, dans tous les cas d'utilisation.

Un troisième but de la présente invention est de fournir un dispositif ne nécessitant pas de brimballement pour obtenir une localisation des désintégrations.

Pour cela, l'invention préconise l'utilisation d'au moins deux scintillateurs disposés en regard et associés à des ensembles de photomultiplicateurs. Des mesures de coïncidence avec détermination des temps de vol sont effectuées en corrélation avec des mesures barycentriques de position des scintillations sur chacun des scintillateurs.

Ces informations permettent de construire une image en volume des désintégrations de positons.

De façon plus précise, l'invention concerne un dispositif pour la visualisation de désintégrations de positons, chaque désintégration étant source d'émissions simultanées de rayonnements gamma selon deux directions opposées comportant :

– au moins une paire de scintillateurs sensibles aux rayonnements gamma et disposés en regard l'un de l'autre, chaque scintillateur présentant une pluralité de faces,

– des ensembles de photomultiplicateurs associés chacun à un scintillateur,

– pour chaque scintillateur, connecté à l'ensemble de photomultiplicateurs associé, un moyen électronique de mesure barycentrique de la position des scintillations apparaissant dans le scintillateur considéré,

– pour chaque paire de scintillateurs, connectés aux ensembles de photomultiplicateurs associés, des moyens électroniques de détermination des temps de vol des rayonnements gamma détectés en coïncidence dans chacun des scintillateurs de la paire considérée,

– un moyen électronique de localisation des désintégrations de positons connecté aux moyens de mesure barycentrique et de détermination des temps de vol,

– un système de visualisation connecté au moyen de localisation.

La mesure barycentrique permet de localiser la position des traces des désintégrations des positons dans un plan parallèle aux faces en regard des scintillateurs, tandis que la mesure du temps de vol permet de localiser les points de désintégrations sur les traces.

De façon avantageuse, pour améliorer la sensibilité du dispositif de l'invention, celui-ci comporte plusieurs paires de scintillateurs en regard associés à des photomultiplicateurs.

Selon un mode de réalisation de l'invention, pour améliorer la résolution spatiale du dispositif, celui-ci comprend en outre un système de reconstruction d'image connecté au moyen de localisation et incluant le système de visualisation, le dispositif de l'invention comportant alors plusieurs paires de scintillateurs ou une seule paire de scintillateurs tournant autour de son axe de symétrie.

Les photomultiplicateurs d'un ensemble sont répartis sur au moins une face du scintillateur correspondant.

Selon un mode de réalisation avantageux les photomultiplicateurs d'un ensemble associé à un scintillateur sont répartis sur plusieurs des faces de ce scintillateur.

Le fait de disposer des photomultiplicateurs sur plusieurs faces des scintillateurs permet d'améliorer la quantité de lumière collectée et donc la résolution spatiale et temporelle (liée à la mesure du temps de vol) du dispositif. Par ailleurs, la multiplication des photomultiplicateurs permet d'obtenir plus précisément la position des scintillations dans un plan parallèle aux faces en regard des scintillateurs.

De manière préférée, les photomultiplicateurs d'un ensemble associé à un scintillateur sont répartis sur toutes les faces de ce scintillateur.

Les photomultiplicateurs absorbent très peu les rayonnements gamma de 511 KeV. On peut donc en disposer sur la face des scintillateurs soumise à ces rayonnements pour améliorer la résolution.

Avantageusement, les scintillateurs sont des prismes réguliers à bases polygonales. Bien entendu, d'autres formes de scintillateurs peuvent être utilisées telles que des cylindres ...

La géométrie adoptée pour les scintillateurs est celle qui permet, pour un encombrement donné de disposer le nombre maximum de photomultiplicateurs sur les bases et éventuellement sur la tranche.

Selon une variante de réalisation, les scintillateurs sont munis d'un moyen pour l'uniformisation de leur sensibilité aux rayonnements gamma en coïncidence.

Selon un mode préféré de réalisation de la précédente variante, le moyen pour l'uniformisation de la sensibilité d'un scintillateur est un collimateur.

Les collimateurs utilisés dans l'invention ne filtrent que les rayonnements gamma présentant un angle d'incidence important avec la surface du scintillateur contrairement au collimateur des gamma caméra qui ne transmet que les rayonnements gamma parallèles à une seule direction ou convergents. En effet, les rayonnements présentant un angle d'incidence important ont d'autant plus de chances d'avoir les rayonnements appariés, émis dans une direction opposée, hors du champ de l'autre scintillateur de la paire qu'ils ont été créés loin du centre du dispositif. En conséquence, les rayonnements gamma émis avec de grands angles d'incidence par rapport aux surfaces des scintillateurs sont à l'origine de la plupart des coïncidences fortuites détectées.

Par contre, étant donné que la localisation des traces est effectuée par des détections gamma en coïncidence (ce qui correspond à une collimation électronique), il est inutile d'utiliser un collimateur matériel du type de ceux utilisés dans les gamma caméras. Au contraire, l'emploi d'un tel collimateur, réduisant la sensibilité du dispositif, serait néfaste.

Selon un mode de réalisation particulier, les scintillateurs sont des cristaux monolithiques de $BaF_2$.

De manière équivalente, on peut utiliser tout autre cristal scintillateur assurant une bonne résolution temporelle grâce à la qualité de sa scintillation lorsqu'il est soumis à des rayonnements gamma.

On entend par cristal scintillateur aussi bien un cristal monolithique qu'une association de cristaux monolithiques, optiquement couplés par des couplants optiques.

En effet, les scintillateurs peuvent être mosaïqués et comprendre, chacun, plusieurs cristaux aptes à émettre une lumière de scintillation à une longueur d'onde de scintillation sous l'effet d'un rayonnement gamma et présentant un certain indice de réfraction, ces cristaux étant fixés les uns aux autres par un matériau d'assemblage possédant un indice de réfraction sensiblement égal à l'indice de réfraction des cristaux et transparent à la longueur d'onde de scintillation.

Pour des scintillateurs mosaïqués, les cristaux sont avantageusement parallélépipédiques et possèdent des faces latérales, les cristaux étant fixés les uns aux autres par leurs faces latérales.

De toute façon, les caractéristiques et avantages de l'invention apparaîtront mieux après la description qui suit donnée à titre explicatif et nullement limitatif. Cette description se réfère aux dessins annexés, sur lesquels :

– la figure 1, déjà décrite et relative à l'art antérieur, représente schématiquement une vue en coupe d'un tomographe à positons,

– la figure 2, déjà décrite et relative à l'art antérieur, représente schématiquement une vue en coupe d'une "gamma caméra",

– la figure 3 représente schématiquement une vue partielle en perspective d'un exemple de dispositif conforme à l'invention,

– la figure 4 représente schématiquement un dispositif selon l'invention,

– la figure 5 représente schématiquement une variante de réalisation d'un dispositif conforme à l'invention,

– la figure 6 représente schématiquement un scintillateur mosaïqué.

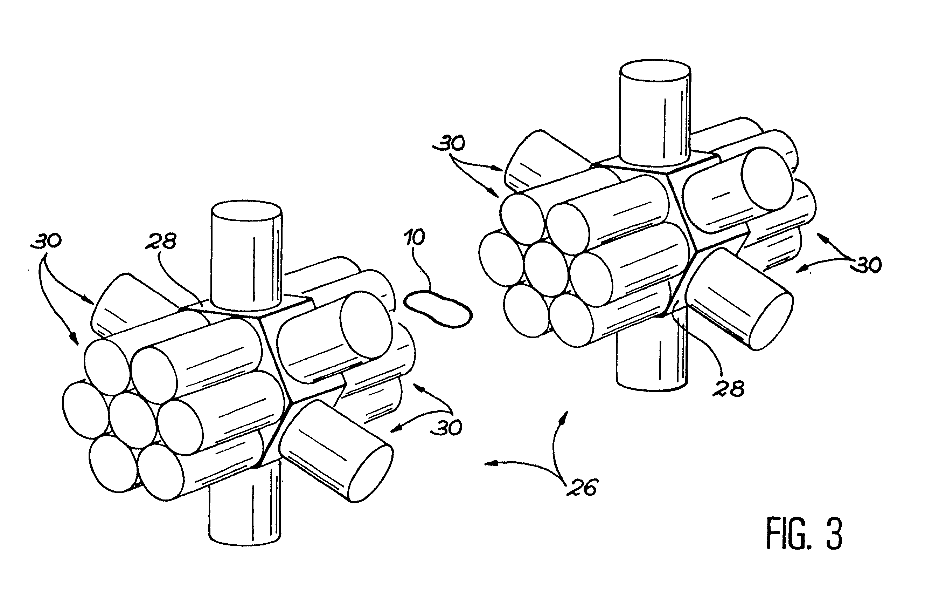

En référence à la figure 3, on décrit maintenant un mode de réalisation des détecteurs d'un dispositif conforme à l'invention. Dans l'exemple de réalisation représenté, le dispositif comporte une paire de détecteurs 26 en regard l'un de l'autre. Ces détecteurs sont disposés de part et d'autre d'un organe 10 marqué par un élément radioactif émettant des positons. Chacun des détecteurs 26 comporte un scintillateur 28 en forme de prisme régulier à bases polygonales, hexagonales dans l'exemple représenté. Les scintillateurs 28 sont par exemple des cristaux monolithiques de $BaF^2$, présentant une largeur entre plats de 240 mm pour une épaisseur de 40 mm.

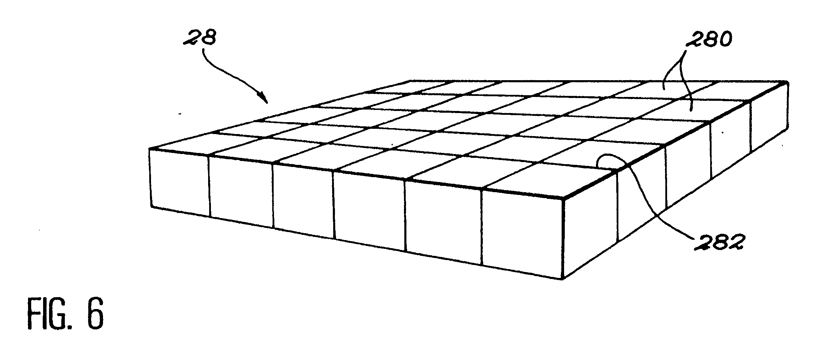

La figure 6 représente schématiquement une variante de réalisation d'un scintillateur. Dans cet exemple de réalisation, le scintillateur 28 est mosaïqué : il comprend plusieurs cristaux 280, par exemple en $BaF^2$ ou CsF, fixés les uns aux autres par leurs faces latérales par un matériau d'assemblage 282.

Le matériau d'assemblage 282 comblant les interstices entre les cristaux possède un indice de réfraction sensiblement égal à l'indice de réfraction des cristaux et est transparent à la longueur d'onde de scintillation. Ce peut être une graisse de silicone, par exemple du type de celle commercialisée par la Société Rhône-Poulenc sous la référence 47-V, une colle à froid, par exemple du type de celle commercialisée par la Société Rhône-Poulenc, par exemple du type de celle commercialisée par la Société Générale Electric sous la référence RTV-615, ou tout autre matériau possédant les caractéristiques adéquates.

Dans l'exemple de la figure 6, le scintillateur mosaïqué comporte 30 cristaux 280 parallélépipédiques de 50 mm de côté et 36 mm d'épaisseur pour constituer un ensemble de 25 cm par 30 cm de côté et de 36 mm d'épaisseur.

Chaque scintillateur 28 est associé à un ensemble de photomultiplicateurs 30 aptes à la détection des scintillations dues aux interactions du scintillateur considéré avec les rayonnements gamma engendrés par les désintégrations des positons.

Suivant le cristal utilisé comme scintillateur dans l'invention, un matériau 20 peut être aussi utilisé entre scintillateur et photomultiplicateur.

De manière avantageuse, les photomultiplicateurs 30, comme les cristaux scintillateurs, présentent un temps de réponse court. On entend par des photomultiplicateurs de temps de réponse court, des photomultiplicateurs dont la fluctuation de temps de transit d'un photo-électron entre photo-cathode et anode a un écart type inférieur à 0,5 ns et par des cristaux à temps de réponse court, des cristaux dont la largeur de l'impulsion lumineuse est inférieure à quelques ns (< ~ 10 ns). Les photomultiplicateurs choisis sont par exemple du type de ceux référencés XP 2020 Q commercialisés par la Société R.T.C.

Dans l'exemple représenté figure 3, chaque face des scintillateurs 28 comporte un ou plusieurs photomultiplicateurs 30 : les bases des scintillateurs 28 comportent dix neuf photomultiplicateurs 30 (dont seuls sept sont représentés) juxtaposés et chaque face de côté comporte un photomultiplicateur 30.

Les photomultiplicateurs étant transparents aux rayonnements gamma de 511 Kev, il n'est pas gênant de placer des photomultiplicateurs sur les faces en regard des scintillateurs 28.

Plus le nombre de photomultiplicateurs est élevé, meilleure est la résolution spatiale. D'autre part, les résolutions tant spatiale que temporelle seront d'autant meilleures que la lumière issue du scintillateur sera mieux collectée par les photomultiplicateurs (c'est-à-dire, que le recouvrement du scintillateur par les photomultiplicateurs sera optimal). En conséquence, pour des géométries de scintillateur et de photomultiplicateur données, on place le plus grand nombre possible de photomultiplicateurs sur les différentes faces du scintillateur.

De façon classique, les détecteurs 26 sont munis de septa latéraux (non représentés), c'est-à-dire de joues protectrices, en plomb par exemple, placées sur les côtés des scintillateurs et interceptant les rayonnements gamma parasites provenant de sources situées hors du champ des détecteurs.

L'élément radioactif se propage dans l'organe 10 et émet des positons qui en se désintégrant émettent deux rayonnements gamma de même énergie (511 keV) mais de directions opposées. Ces rayonnements gamma sont émis dans toutes les directions de l'espace. Lorsqu'un rayonnement pénètre dans un scintillateur, il engendre une scintillation détectée par les photomultiplicateurs. L'épaisseur des scintillateurs est telle que la plupart des scintillations est détectée par au moins deux photomultiplicateurs. Ces derniers délivrent un signal proportionnel à l'intensité de la lumière détectée (quantité de photons détectée).

L'étude des coïncidences, c'est-à-dire des détections effectuées par les scintillateurs appariés et l'étude des temps de vol, effectuée parallèlement à la détermination de la position de l'apparition des scintillations dans les scintillateurs permettent de localiser les points de désintégration des positons.

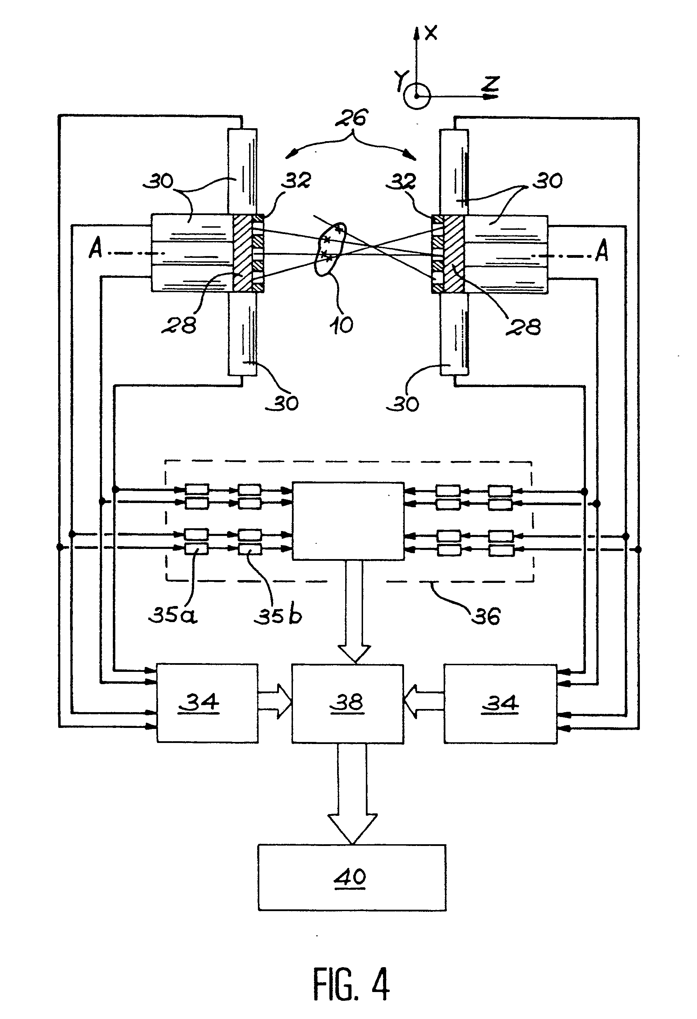

En référence à la figure 4, on décrit maintenant une variante de réalisation d'un dispositif conforme à l'invention.

Dans cette réalisation, le dispositif comporte une paire de détecteurs 26 en regard l'un de l'autre représentés en coupe. Les photomultiplicateurs 30 sont disposés sur les scintillateurs 28 de manière identique à la réalisation précédente (figure 3), à ceci près que les bases en regard des scintillateurs 28 en sont dépourvues. Par contre, ces bases comportent un collimateur 32 composé d'une plaque de matériau absorbant par exemple de plomb percée de trous. Si des photomultiplicateurs sont couplés sur les faces internes des cristaux (voir figure 3) le collimateur peut être placé entre ces photomultiplicateurs et l'organe 10. Les collimateurs 32 présentent un angle d'acceptance d'une vingtaine de degrés environ ; c'est-à-dire que les rayonnements gamma ayant une incidence supérieure à la moitié de cet angle sont interceptés par la plaque de matériau absorbant.

Il en résulte une diminiation sensible des coïncidences provenant de points source de rayonnements au centre du champ visuel en coïncidence des détecteurs pour lequels les angles d'incidence peuvent être élevés, par contre l'effet du collimateur est très peu sensible pour les coïncidences provenant des bords du champ visuel, lesquelles sont issues des rayonnements à petit angle d'incidence.

En conséquence, la réponse du système est uniformisée en sensibilité entre centre et bord du champ visuel.

Le rôle du collimateur est également de supprimer les évènements uniques arrivant sous grand angle d'incidence ce qui améliore le rapport évènements fortuits/événements utiles.

Chaque ensemble de photomultiplicateurs 30 associé à un scintillateur 28 est connecté à un moyen 34 électronique de mesure barycentrique de la position des scintillations apparaissant dans le scintillateur considéré. Ce type de moyen électronique est par exemple décrit dans le brevet US n° 3 011 057 et il n'est donc pas utile de reprendre le détail de cette description. Il suffit de savoir que les scintillations sont positionnées par rapport à un axe X et un axe Y définissant un plan parallèle à une des bases du scintillateur considéré. Ces axes X et Y se croisent à hauteur du centre de la base du scintillateur. Ils sont chacun divisés en une partie de coordonnées positives X+, Y+ et une partie de coordonnées négatives X-, Y-. Le courant débité pour chaque photomultiplicateur 30 de l'ensemble traverse quatre résistances dont les valeurs dépendent de la position du photomultiplicateur par rapport aux axes X et Y. On détermine le barycentre du point d'apparition d'une scintillation par addition des différents courants (pondérés par l'intensité détectée par chaque photomultiplicateur) issus des photomultiplicateurs excités.

Les moyens 34 de mesure barycentrique comportent des moyens de compensation d'asynchronisme dû à la position du point d'impact du rayonnement gamma sur le scintillateur 28. En effet, le temps de parcours de la lumière entre son point de création et chacun des photomultiplicateurs est fonction de la localisation du point d'impact des rayonnements gamma.

Les moyens 34 de mesure barycentrique délivrent des signaux proportionnels à la position en X et Y des scintillations apparaissant dans chacun des scintillateurs 28.

Parallèlement à ces mesures barycentriques, des moyens 36 électroniques de détermination de temps de vol des rayonnements gamma détectés en coïncidence dans chacun des scintillateurs 28 sont connectés aux deux ensembles de photomultiplicateurs 30. De tels moyens 36 sont par exemple décrits dans le rapport "Workshop on time of flight tomography", 17-19 May 1982, Washington University, Saint-Louis, Missouri, p.143-146 ; il n'est donc pas utile de reprendre le détail de cette description. Il suffit de savoir que les signaux issus des photomultiplicateurs 30 associés à chacun des scintillateurs 28 et délivrés en coïncidence, c'est-à-dire dans une fenêtre temporelle déterminée, sont tout d'abord mis en forme par un discriminateur à fraction constante qui délivre des impulsions calibrées. Ces impulsions sont délivrées en entrée d'un convertisseur de temps en amplitude d'un signal électrique. Cette amplitude correspond à la charge d'un condensateur à courant constant et donc à droite de charge linéaire. La charge débute à l'arrivée d'une première impulsion et est stoppée à l'arrivée de la suivante provenant du rayonnement détecté en coïncidence. Cette amplitude est ensuite convertie en une valeur binaire par un convertisseur analogique/digital. Cette valeur correspond au temps de vol et est associée aux coordonnées X, Y des interactions gamma correspondantes sur chaque scintillateur.

Les moyens 36 de détermination de temps de vol comprennent en outre des moyens 35a de traitement des signaux délivrés par les photomultiplicateurs qui compensent les différences de temps de réponse des photomultiplicateurs. Une phase préalable de calibration permet d'évaluer le retard individuel de chaque photomultiplicateur.

Les moyens 36 comportent en outre des moyens 35b de compensation d'asynchronisme dû à la position du point d'impact du rayonnement gamma sur le scintillateur 28.

Les adresses des photomultiplicateurs ont déjà été déterminées par les moyens 34 et correspondent aux coordonnées en X et Y.

Les signaux délivrés en sortie des moyens 34, 36 de mesure barycentrique et de détermination des temps de vol sont appliqués en entrée d'un moyen 38 électronique de localisation des désintégrations de positons qui associe les valeurs des temps de vol aux coordonnées XY calculées. L'écart entre les scintillateurs 28 de la paire étant connu, les données des positions des scintillations détectées en coïncidence et des temps de vol permettent de calculer la localisation dans l'espace (par rapport à un repère de référence) des points de désintégration de positons.

Le moyen 38 de localisation délivre sur une sortie les coordonnées des points de désintégration par rapport au repère de référence. Cette sortie est connectée à un moyen 40 de visualisation qui permet d'obtenir sur un écran par exemple les différentes densités de désintégration de positons dans l'espace contenu entre les deux détecteurs 26. Pour améliorer la résolution spatiale du dispositif, ce moyen de visualisation peut être remplacé par un moyen de reconstruction et de visualisation d'image. Dans ce cas particulier, les détecteurs 26 sont animés d'un mouvement de rotation autour de l'axe de symétrie X ou Y, à mi-distance entre les détecteurs 26.

Le moyen de reconstruction d'image est constitué de façon connue par des moyens faisant appel à des traitements tels que des projections, des rétroprojections, des convolutions ... utilisés classiquement dans les tomographes à temps de vol comme le TTV03 du D.LETI.

Ce moyen est décrit par exemple dans les documents : - R.Allemand et al : Diagnostic Imaging Medecine PISA (Italy) Oct.11-24 1981 ; - Ph.Garderet, E.Campagnolo : Image Reconstruction Using Time of Flight Information in the Leti Positon Tomography System - Workshop on Time-OF-Flight Tomography, May 19, 1982, St Louis, Missouri, p.97.

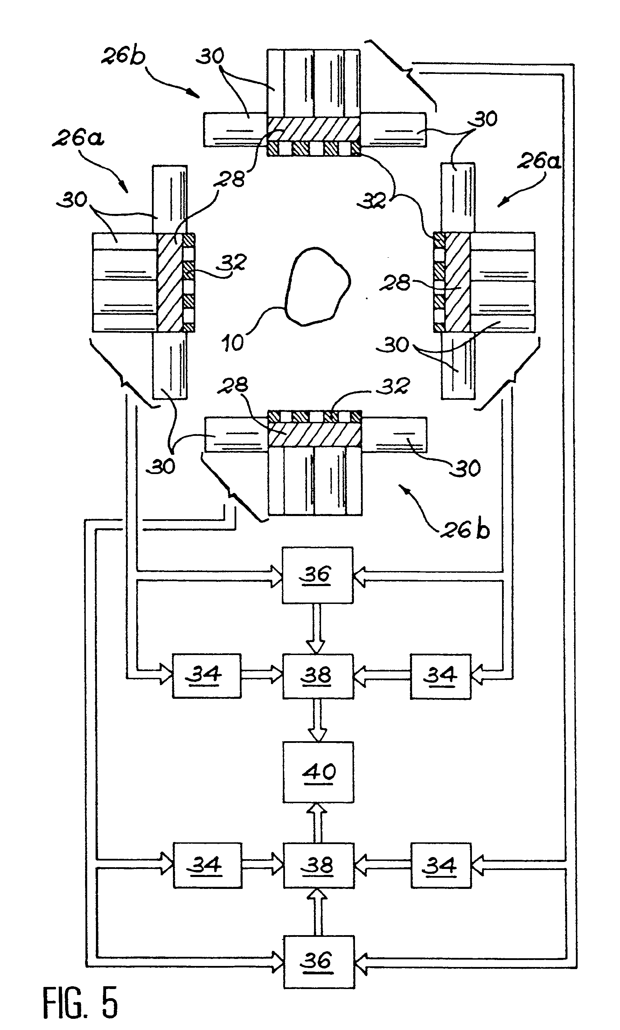

La figure 5 représente schématiquement une autre variante de réalisation d'un dispositif conforme à l'invention. Ce dispositif comporte deux paires de détecteurs 26a, 26b. Les scintillateurs 28 des détecteurs d'une paire sont en regard l'un de l'autre et les deux paires sont decalées de 90° l'une de l'autre. Les moyens électroniques 34 de mesure barycentrique, 36 de temps de vol, et 38 de localisation des désintégrations sont doublés et correspondent chacun à une paire de détecteurs.

Le moyen de visualisation 40 est connecté aux deux moyens 38 de localisation des désintégrations de positons, ce qui permet d'obtenir un dispositif ayant une meilleure sensibilité.

Le moyen de visualisation 40 peut être remplacé par un moyen de reconstruction et de visualisation d'image, la reconstruction d'image permettant d'améliorer la résolution spatiale, ces paires de détecteurs pouvant ou non tourner auotur de l'organe 10.

Bien entendu, un dispositif conforme à l'invention ne se limite pas aux exemples de réalisation plus particulièrement décrit ; il en admet au contraire toutes les variantes. En particulier, on peut réaliser un dispositif conforme à l'invention avec plus de deux paires de scintillateurs.

## Revendications

1. Dispositif pour la visualisation de désintégrations de positons, chaque désintégration étant source d'émissions simultanées de rayonnements gamma, caractérisé en ce qu'il comporte :
   – au moins une paire de scintillateurs (28) sensibles aux rayonnements gamma et disposés en regard l'un de l'autre, chaque scintillateur (28) présentant une pluralité de faces,
   – des ensembles de photomultiplicateurs (30) associés chacun à un scintillateur (28),
   – pour chaque scintillateur (28), connecté à l'ensemble de photomultiplicateurs (30) associé, un moyen électronique (34) de mesure barycentrique de la position des scintillations apparaissant dans le scintillateur considéré (28),
   – pour chaque paire de scintillateurs (28), connectés aux ensembles de photomultiplicateurs (30) associés, des moyens électroniques (36) de détermination des temps de vol des rayonnements gamma détectés en coïncidence dans chacun des scintillateurs (28) de la paire considérée,
   – un moyen électronique (38) de localisation des désintégrations de positons connecté aux moyens de mesure barycentrique et de détermination des temps de vol,
   – un système (40) de visualisation connecté au moyen de localisation.

2. Dispositif selon la revendication 1, caractérisé en ce qu'il comprend plusieurs paires de scintillateurs (28) en regard associés à des photomultiplicateurs.

3. Dispositif selon la revendication 1, caractérisé en ce qu'il comprend en outre un système de reconstruction d'image connecté au moyen de localisation et incluant le système de visualisation.

4. Dispositif selon la revendication 3, caractérisé en ce qu'il comprend une paire de scintillateurs tournant autour de son centre de symétrie.

5. Dispositif selon la revendication 1, caractérisé en ce que les photomultiplicateurs (30) d'un ensemble associé à un scintillateur (28) sont répartis sur au moins une face de ce scintillateur (28).

6. Dispositif selon la revendication 5, caractérisé en ce que les photomultiplicateurs (30) d'un ensemble associé à un scintillateur (28) sont répartis sur toutes les faces de ce scintillateur (28).

7. Dispositif selon la revendication 1, caractérisé en ce que les scintillateurs (28) sont des prismes réguliers à bases polygonales.

8. Dispositif selon la revendication 1, caractérisé en ce que les scintillateurs (28) sont munis d'un moyen (32) pour l'uniformisation de leur sensibilité aux rayonnements gamma.

9. Dispositif selon la revendication 8, caractérisé en ce que le moyen (32) pour l'uniformisation de la sensibilité d'un scintillateur est un collimateur.

10. Dispositif selon la revendication 1, caractérisé en ce que les scintillateurs (28) sont des cristaux monolithiques de $BaF_2$.

11. Dispositif selon la revendication 1, caractérisé en ce que les scintillateurs (28) sont mosaïqués et comprennent, chacun, plusieurs cristaux aptes à émettre une lumière de scintillation à une longueur d'onde de scintillation sous l'effet d'un rayonnement gamma et présentant un certain indice de réfraction, ces cristaux étant fixés les uns aux autres par un matériau d'assemblage possédant un indice de réfraction sensiblement égal à l'indice de réfraction des cristaux et transparent à la longueur d'onde de scintillation.

12. Dispositif selon la revendication 11, caractérisé en ce que les cristaux sont parallélépipédiques et possèdent des faces latérales, les cristaux étant fixés les uns aux autres par leurs faces latérales.

FIG. 1

FIG. 2

FIG. 3

FIG. 4

FIG. 5

FIG. 6

**Office européen des brevets**

**RAPPORT DE RECHERCHE EUROPEENNE**

Numero de la demande

EP 91 40 0663

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| A | US-A-4 150 292 (TER-POGASSIAN) <br> * Abrégé; colonne 9, ligne 24 - colonne 10, ligne 63; figures * <br> --- | 1-5,8,9 | G 01 T 1/29 <br> G 01 T 1/202 |
| A | EP-A-0 006 900 (COMMISSARIAT A L'ENERGIE ATOMIQUE) <br> * Abrégé; page 7, ligne 27 - page 8, ligne 13; figure 1 * <br> --- | 1,3,10 | |
| A | US-A-4 755 680 (LOGAN) <br> * Abrégé; colonne 6, lignes 41-52; colonne 7, ligne 39 - colonne 8, ligne 31; figures * <br> --- | 1 | |
| A | EP-A-0 212 631 (CLAYTON FOUNDATION FOR RESEARCH) <br> * Abrégé; page 10, ligne 26 - page 11, ligne 27; figure 5 * <br> ----- | 11,12 | |
| | | | **DOMAINES TECHNIQUES RECHERCHES (Int. Cl.5)** |
| | | | G 01 T |

Le présent rapport a été établi pour toutes les revendications

| Lieu de la recherche | Date d'achèvement de la recherche | Examinateur |
|---|---|---|
| LA HAYE | 08-07-1991 | DATTA S. |

**CATEGORIE DES DOCUMENTS CITES**

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

        ................................................................
& : membre de la même famille, document correspondant

EPO FORM 1503 03.82 (P0402)